# EUROPÄISCHE PATENTSCHRIFT

(11) **EP 0 528 177 B1**
(45) Veröffentlichungstag und Bekanntmachung des Hinweises auf die Patenterteilung: **11.09.1996**
(21) Anmeldenummer: 92112132.3
(22) Anmeldetag: 16.07.1992
(51) Int. Cl.: A61B 17/58

(54) **Fixateur interne zur Reposition einer lumbalen Spondylolisthesis**
Internal fixator for the correction of a lumbar spondyldisthesis
Fixateur externe pour la correction d'une spondylolisthésis lombaire

(30) Priorität: 17.08.1991 DE 4127303
(43) Veröffentlichungstag der Anmeldung: 24.02.1993
(73) Patentinhaber: AESCULAP AG, D-78501 Tuttlingen (DE)
(72) Erfinder: Blömer, Wilhelm, W-7200 Tuttlingen (DE); Braun, Karl, W-7201 Talheim (DE)
(74) Vertreter: Böhme, Ulrich Hoeger, Stellrecht & Partner

(56) Entgegenhaltungen:
- WO-A-90/02527
- DE-U- 9 110 203
- US-A- 1 920 821
- UNFALLCHIRURGIE Bd. 12, Nr. 2, 1986, Seiten 68 - 79 P.KLUGER UND H.J.GERNER 'Das mechanische Prinzip des Fixateur externe zur dorsalen Stabilisierung der Brust- und Lendenwirbelsäule'

## Beschreibung

Die Erfindung betrifft einen Fixateur interne zur Reposition einer lumbalen Spondylolisthesis mit zwei in den zu reponierenden Wirbelkörper beziehungsweise einen benachbarten Knochen einschraubbaren Knochenschrauben, die in ihren aus den Knochen hervorstehenden Bereichen mittels einer Querstrebe gelenkig verbunden sind, mit an den Knochenschrauben angreifenden, sich in deren Längsrichtung erstreckenden Verlängerungen und mit einer Spannvorrichtung, die eine der beiden Knochenschrauben mit einer Zugkraft beaufschlagt, wobei die Spannvorrichtung einen drehbar an der Verlängerung der einen Knochenschraube gelagerten Schwenkhebel aufweist, der im wesentlichen quer zur Längsrichtung der Verlängerung verläuft.

Aus der deutschen Patentschrift 36 25 542 ist ein Fixateur interne bekannt, bei dem die Spannvorrichtung durch eine Gewindespindel gebildet ist, die parallel zu der Knochenschraube verläuft, auf die die Zugkraft ausgeübt werden soll. Die Spannvorrichtung stützt sich dabei an der Querstrebe ab, die auf diese Weise die notwendigen Kräfte zur Reposition des Wirbelkörpers von der Spannvorrichtung auf die andere Knochenschraube und den benachbarten Knochen übertragen muß. Es ergibt sich dadurch eine Konstruktion, die entweder die notwendigen Kräfte nicht starr in den benachbarten Knochen einleiten kann oder die so kräftig gebaut werden muß, daß sich ein sperriger und für die Handhabbarkeit zu schwerer Aufbau ergibt.

Aus der internationalen Patentanmeldung WO 90/02527 ist ein gattungsgemäßer Fixateur interne bekannt, bei dem die Spannvorrichtung zwei Gewindestangen umfaßt, die an beiden Verlängerungen der Knochenschrauben drehbar gelagert sind. Soll eine Reposition erzielt werden, so müssen beide Gewindestangen so verdreht werden, daß sie schließlich zusammen mit den Verlängerungen ein Rechteck bilden. Die Einleitung der erforderlichen Zugkräfte ist somit recht aufwendig, und in der Handhabung ist dieser Fixateur interne schwierig.

Es ist Aufgabe der Erfindung, einen gattungsgemäßen Fixateur interne so zu verbessern, daß die Einleitung der zur Reposition des Wirbelkörpers notwendigen Zugkräfte in den benachbarten Knochenteil verbessert wird.

Diese Aufgabe wird bei einem Fixateur interne der eingangs beschriebenen Art erfindungsgemäß dadurch gelöst, daß der Schwenkhebel an einem Anschlag der anderen, dem zu reponierenden Wirbelkörper zugeordneten Verlängerung anliegt, und die Gelenkverbindungen zwischen Knochenschrauben und Querstrebe fixierbar sind.

Bei einer solchen Lösung wird die Zugkraft durch den Schwenkhebel übertragen. Dieser ist an der Verlängerung gelagert, die dem nicht zu reponierenden Knochenteil zugeordnet ist, hier erfolgt also die Einleitung der Zugkräfte aus der Lagerstelle des Schwenkhebels unmittelbar in den nicht zu reponierenden Knochenteil, der somit ein Widerlager für die gesamte Vorrichtung bildet. Dabei kann es sich beispielsweise um einen dem zu reponierenden Wirbelkörper benachbarten Wirbelkörper handeln. Insbesondere ist es bei dieser Konstruktion nicht notwendig, daß die Querstrebe als Widerlager für eine Spannvorrichtung dient, so daß bei der erfindungsgemäßen Konstruktion beim Reponieren des Wirbelkörpers die Schwenkverbindungen zwischen Querstrebe und Verlängerungen gelöst bleiben können.

Bei einer bevorzugten Ausführungsform ist vorgesehen, daß der Anschlag an der Verlängerung des zu reponierenden Wirbelkörpers um eine Achse verschwenkbar ist, die zur Schwenkachse des Hebels parallel verläuft. Vorzugsweise umgibt dabei der Anschlag den Hebel und ist relativ zu dem Hebel in dessen Längsrichtung frei verschieblich. Man erhält auf diese Weise eine verschwenkbare Lagerhülse für den Hebel, die den Hebel einerseits an der Verlängerung des zu reponierenden Wirbelkörpers führt und andererseits die Zugkräfte auf die Verlängerung überträgt, ohne die Verschwenkung der Verlängerung beim Distrahieren der Wirbel zu behindern.

Es kann vorgesehen sein, daß der Hebel im Bereich zwischen den beiden Verlängerungen ein Außengewinde trägt, auf das eine Anschlagmutter aufgeschraubt ist. Damit wird die effektive Länge des Hebels zwischen der Anlenkstelle desselben und dem Anschlag der Verlängerung festgelegt, so daß beim Verschwenken des Hebels die Querstrebe und der Hebel gemeinsam mit den Verlängerungen eine parallelogrammlenkerähnliche Führung bilden, die eine im wesentlichen kippungsfreie Querverschiebung des zu reponierenden Wirbelkörpers ermöglichen.

Günstig ist es weiterhin, wenn der Hebel zweiarmig ausgebildet ist, wobei ein Arm zu dem Anschlag an der Verlängerung des zu reponierenden Wirbelkörpers führt, während der andere als Griffteil ausgeführt ist. Durch eine solche zweiarmige Ausgestaltung des Hebels kann der Operateur den Hebel mit Hilfe des Griffteils sehr gefühlvoll verschwenken und dabei unter Umständen auch über eine größere Übersetzung die notwendige Kraft zur Querverschiebung des zu reponierenden Wirbelkörpers aufbringen.

Es kann weiterhin vorgesehen sein, daß die Verlängerung, an der der Hebel gelagert ist, einen festen Anschlag trägt, gegen den ein am Hebel verstellbar gelagertes Stellglied anschlägt und dadurch ein Absinken des zu reponierenden Wirbelkörpers verhindert. Eine einmal erreichte Repositionsstellung kann auf diese Weise fixiert werden, beispielsweise um eine genaue Kontrolle der erreichten Wirbelposition zu ermöglichen.

Das Stellglied ist vorzugsweise eine in ein Innengewinde des Hebels eingeschraubte, aus dem Innengewinde hervorstehende Stellschraube.

Bei einer besonders bevorzugten Ausführungsform ist vorgesehen, daß die Verlängerungen lösbar mit den Knochenschrauben verbunden sind. Im Gegensatz zu herkömmlichen Verlängerungen, die am Ende der Operation abgeknipst werden, lassen sich die Verlängerungen einfach abnehmen. Sie können auch zu einem späteren Zeitpunkt wieder aufgesetzt werden, so daß die Reposition unter Umständen auch in Etappen vorgenommen werden kann. Scharfe Stellen, die durch das Abzwicken der Verlängerungen bei herkömmlichen Verlängerungen entstehen und unter Umständen zu Verletzungen führen könnten, sind dadurch mit Sicherheit vermieden.

Insbesondere können die Verlängerungen Hülsen aufweisen, an denen der Hebel beziehungsweise der Anschlag gelagert ist. Diese Hülsen sind durch die Hülsen durchsetzende, in die Knochenschrauben einschraubbare Fixierschrauben an den Knochenschrauben gehalten.

Die Verlängerungen können zusätzlich an ihren freien Enden Rücksprünge zur Aufnahme eines Distraktionsinstrumentes tragen, mit dessen Hilfe vor der eigentlichen Reposition eine Distraktion der Wirbelsäule erfolgt, um dem zu reponierenden Wirbelkörper überhaupt den Platz zu schaffen, der für eine Reposition benötigt wird.

Die nachfolgende Beschreibung bevorzugter Ausführungsformen der Erfindung dient im Zusammenhang mit der Zeichnung der näheren Erläuterung. Es zeigen:
- Figur 1 :: ein in die Wirbelsäule eingeschraubter Fixateur interne mit Repositionsinstrument vor der Reposition des Wirbelkörpers;
- Figur 2 :: eine Ansicht ähnlich Figur 1 nach der Reposition des Wirbelkörpers und
- Figur 3 :: eine perspektivische Ansicht der die Repositionsvorrichtung bildenden Teile vor dem Zusammenbau.

Das in der Zeichnung dargestellte chirurgische Instrument umfaßt einen Fixateur interne mit einer aufgesetzten Repositionseinrichtung, die abnehmbar ausgestaltet ist. Zur Reposition eines Wirbelkörpers, der sich seitlich gegenüber der Längsachse der Wirbelsäule verschoben hat, werden zwei derartige Instrumente benötigt, die zu beiden Seiten der Wirbelsäulen-Längsebene symmetrisch zueinander angeordnet werden. Diese Instrumente können vollständig gleich oder aber genau spiegelbildlich ausgebildet werden, so daß in der folgenden Beschreibung nur eines dieser Instrumente erörtert wird.

Jedes dieser Instrumente umfaßt zwei im wesentlichen gleich ausgebildete Knochenschrauben 1, 2 mit einem Einschraubgewinde 3 beziehungsweise 4 und mit einem sich daran anschließenden glatten Schaft 5 beziehungsweise 6, der jeweils von einem flanschartig überstehenden Bund 7, 8 begrenzt wird.

Auf den Schaft 5, 6 jeder Knochenschraube 1 beziehungsweise 2 ist je ein Klemmstück 9 beziehungsweise 10 aufgeschoben. Die beiden Klemmstücke 9 und 10 werden von einer als Gewindestab ausgebildeten Querstrebe 11 durchdrungen, die im wesentlichen quer zur Längsrichtung der Knochenschrauben 1 und 2 verläuft.

Die Querstrebe 11 ist in den Klemmstücken 9, 10 eingeschraubt und dadurch in axialer Richtung festgelegt. Beide Klemmstücke 9 und 10 weisen Klemmschrauben 12 und 13 auf, die in gelöstem Zustand eine Verschwenkung der Querstrebe 11 gegenüber den Knochenschrauben 1, 2 ermöglicht, und zwar um eine Achse, die senkrecht auf der durch die Querstrebe und die Knochenschrauben aufgespannten Ebene liegt. Durch Festspannen der Klemmschrauben 12 und 13 können die Klemmstücke 9 und 10 so festgelegt werden, daß diese Verschwenkung der Querstrebe 11 gegenüber den Knochenschrauben 1 und 2 unterbunden wird.

Beide Knochenschrauben 1, 2 weisen an ihrem dem Einschraubgewinde 3 beziehungsweise 4 abgewandten Ende eine Innengewindebohrung 14 beziehungsweise 15 auf, in die je eine Verlängerung 16 beziehungsweise 17 eingeschraubt ist. Beide Verlängerungen weisen eine längliche Hülse 18 beziehungsweise 19 auf, durch die eine Fixierschraube 20, 21 hindurchgreift, die in die Innengewindebohrungen 14 beziehungsweise 15 der Knochenschrauben 1 beziehungsweise 2 eingeschraubt sind. Die Hülsen 18, 19 tragen in ihrem oberen Endbereich Riffelungen 22 beziehungsweise 23, ebenso sind an den oberen Enden der Fixierschrauben 20, 21 in dem aus den Hülsen 18 beziehungsweise 19 hervorstehenden Bereichen Riffelungen 24 beziehungsweise 25 angeordnet.

An den freien Enden der Fixierschrauben 20 und 21 tragen diese einen stiftförmigen Fortsatz 26 beziehungsweise 27 mit zwei parallelen Bünden 28, 29 beziehungsweise 30, 31. Die zwischen diesen Bünden ausgebildeten Nuten oder Rücksprünge 32, 33 beziehungsweise 34 und 35 dienen einem in der Zeichnung nicht dargestellten, an sich bekannten Distraktionsinstrument als Angriffsfläche. Ein solches Distraktionsinstrument kann beispielsweise in die Nuten einlegbare Haken tragen, die an den Branchen des zangenartig ausgebildeten Instrumentes gehalten sind.

Soweit die Teile des Fixateur interne bisher beschrieben worden sind, sind diese vollständig symmetrisch ausgebildet, das heißt die Knochenschrauben, die in die eingesetzten Verlängerungen und die auf den Knochenschrauben gehaltenen Klemmstücke sind in beiden Fällen gleich ausgebildet; diese gleich ausgebildeten Baueinheiten werden durch die Querstrebe miteinander verbunden.

Im folgenden ergeben sich jedoch Unterschiede der weiteren Ausgestaltung dieser Baueinheiten.

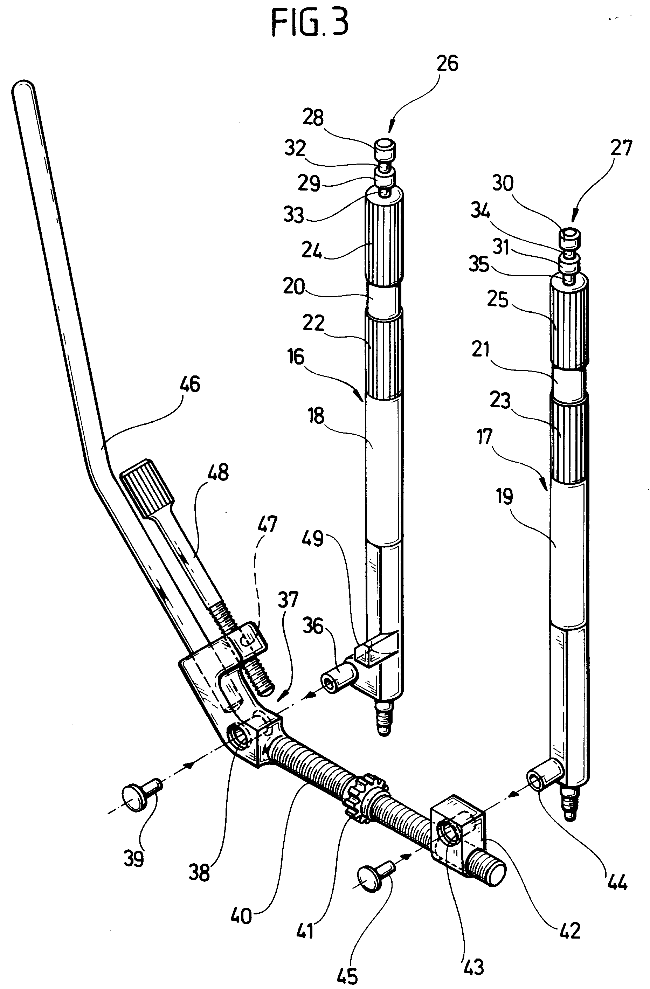

An einer Hülse 18 ist an deren unterem Rand ein senkrecht abstehender Lagerbolzen 36 angeordnet, auf dem ein zweiarmiger Hebel 37 verschwenkbar gelagert ist. Zur Festlegung auf dem Lagerbolzen 26 wird eine eine Lagerbohrung 38 am Hebel 37 durchsetzende Schraube 39 verwendet (Figur 3).

Ein Arm 40 des Hebels 37 ist als Gewindestange ausgebildet, auf welche eine Rändelmutter 41 aufgeschraubt ist. Der Arm 40 taucht weiterhin in eine Lagerhülse 42 ein, in der der Arm 40 in Längsrichtung frei verschieblich ist. Die Lagerhülse 42 weist eine Lagerbohrung 43 auf, diese wiederum ist auf einem Lagerbolzen 44 verdrehbar gelagert, der parallel zum Lagerbolzen 36 angeordnet ist, jedoch an der Hülse 19 der anderen Verlängerung. Festgelegt ist die Lagerhülse 42 durch eine in den Lagerbolzen 44 eingedrehte Schraube 45.

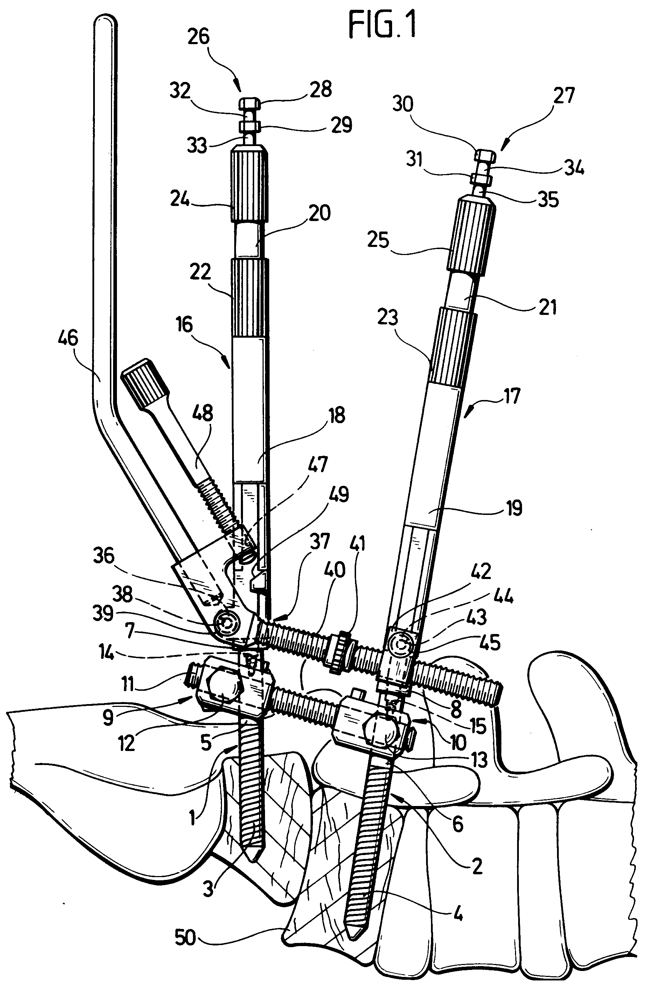

Der andere Arm 46 des Hebels 37 schließt mit dem Arm 40 einen Winkel von etwa 135° ein, er ist seinerseits abgewinkelt, so daß das freie Ende des Armes 46 etwa in Richtung der Knochenschrauben und der Verlängerungen weist (Figur 1).

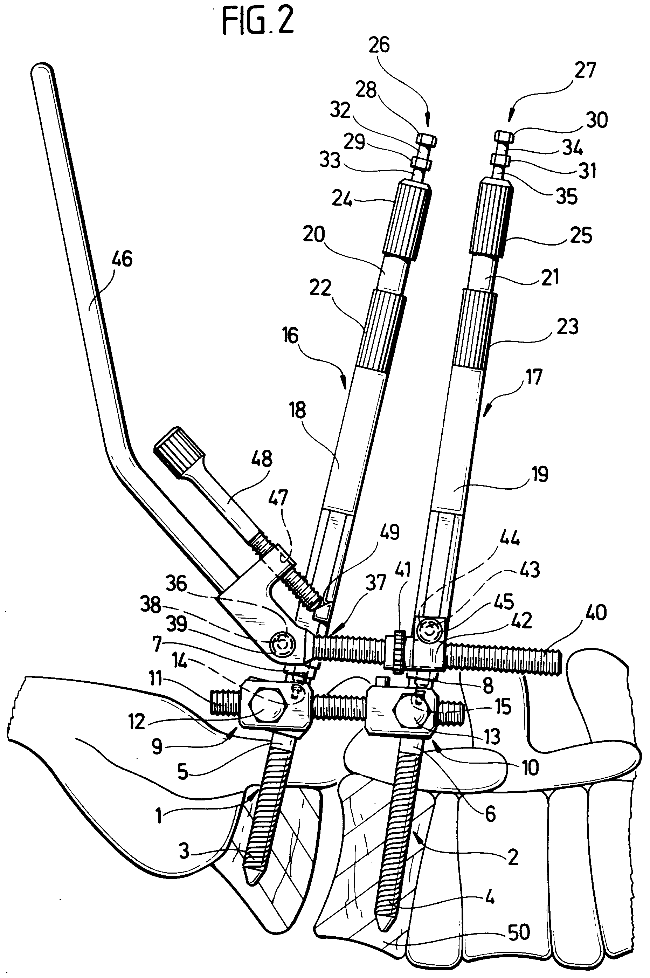

In ein Innengewinde 47 des Hebels 37, das im wesentlichen parallel zum Arm 46 verläuft, ist eine Stellschraube 48 eingedreht, die mit ihrem freien Ende an einen festen Anschlag 49 der Hülse 18 anlegbar ist. Wenn die Stellschraube 48 am Anschlag 49 anliegt, ist eine Verschwenkbewegung des Hebels 37 in einer Richtung blockiert, im Ausführungsbeispiel der Figuren 1 und 2 in Uhrzeigerrichtung.

Bei der Verwendung des beschriebenen Instrumentes werden zunächst die beiden Knochenschrauben 1 und 2 eingesetzt, und zwar eine Knochenschraube in den zu reponierenden Wirbelkörper 50, die andere Knochenschraube in einen benachbarten Knochen, beispielsweise einen benachbarten Wirbelkörper oder in den Hüftknochen. Das Einschrauben erfolgt dabei seitlich gegenüber der Längsmittelebene der Wirbelsäule versetzt, zu beiden Seiten dieser Längsmittelebene werden gleich ausgebildete Instrumente eingesetzt.

Nach dem Einschrauben der Knochenschrauben 1 und 2 werden die Klemmstücke 9 und 10 mit der Querstrebe 11 auf die Schäfte 5 und 6 der Knochenschrauben 1 beziehungsweise 2 aufgesetzt. Die Klemmstücke 9 und 10 bleiben gelockert, das heißt die Querstrebe ist gegenüber den Knochenschrauben 1 und 2 verschwenkbar, jedoch wird der Abstand der beiden Klemmstücke 9 und 10 durch die Querstrebe 11 festgelegt.

Mittels der Fixierschrauben 20 und 21 werden anschließend die Hülsen 18 beziehungsweise 19 auf die Knochenschrauben 1 beziehungsweise 2 aufgesetzt und dort fixiert. An den beiden Hülsen 18 und 19 greift in der beschriebenen Weise der zweiarmige Hebel 37 an. Diese Ausgangslage der Operation ist in Figur 1 dargestellt. Die Stellschraube 48 ist dabei vom Anschlag 49 entfernt, die Rändelmutter 41 steht im Abstand zur Lagerhülse 42.

In einem ersten Arbeitsschritt wird die Wirbelsäule durch Distraktion gedehnt. Dazu wird an den stiftförmigen Fortsätzen 26 und 27 der beiden Hülsen 18 beziehungsweise 19 angegriffen und die Enden dieser Hülsen 18 und 19 werden mittels eines geeigneten Distraktionsinstrumentes einander angenähert. Aufgrund des durch die Querstrebe 11 festgelegten Abstandes der aus Knochenschraube und Verlängerung bestehenden Baueinheiten führt dies einerseits zu einem Aufspreizen der Wirbelsäule und andererseits zu einer leichten Kippung des zu reponierenden Wirbelkörpers 50. Da die Klemmstücke 9 und 10 nicht festgelegt sind, lassen sich die beiden Baueinheiten in geringem Umfange gegeneinander verschwenken. Der Hebel 37 behindert diese Distraktion in keiner Weise, bei der Distraktion gleitet der Arm 40 in der Lagerhülse 42, die ihre Winkellage gegenüber der Hülse 19 frei einstellen kann.

Nach der Distraktion erfolgt die eigentliche Reposition des Wirbelkörpers 50 durch eine Verschwenkung des zweiarmigen Hebels 37 in der Weise, daß der Arm 40 eine nach oben gerichtete Zugkraft auf die Hülse 19 und damit die Knochenschraube 2 ausübt. Dies läßt sich einfach durch Verschwenken des Hebels im Gegenuhrzeigersinn (Figuren 1 und 2) erreichen, wobei die Lagerkräfte für den Hebel 37 unmittelbar über die Hülse 18 und die Knochenschraube 1 in den als Widerlager wirkenden Knochen eingeleitet werden.

Sobald die gewünschte Wirbellage erreicht ist, kann diese durch Anlegen der Stellschraube 48 am Anschlag 49 und durch Anlegen der Rändelmutter 41 an der Lagerhülse 42 momentan fixiert werden. Anschließend werden die Klemmschrauben 12 und 13 gespannt, so daß die beiden Knochenschrauben 1 und 2 und die Querstrebe 11 einen nunmehr starren Fixateur für den zu reponierenden Wirbelkörper 50 bilden. Danach können die Verlängerungen 16 und 17 durch Lösen der Fixierschrauben 20 und 21 von den Knochenschrauben 1 und 2 abgenommen werden, so daß im Körper lediglich der aus den Knochenschrauben 1 und 2 und der Querstrebe 11 gebildete Fixateur interne verbleibt.

## Patentansprüche

1. Fixateur interne zur Reposition einer lumbalen Spondylolisthesis mit zwei in den zu reponierenden Wirbelkörper beziehungsweise einen benachbarten Knochen einschraubbaren Knochenschrauben (1, 2), die in ihren aus den Knochen hervorstehenden Bereichen mittels einer Querstrebe (11) gelenkig verbunden sind, mit an den Knochenschrauben (1, 2) angreifenden, sich in deren Längsrichtung erstreckenden Verlängerungen (16, 17) und mit einer Spannvorrichtung, die eine der beiden Knochenschrauben (1, 2) mit einer Zugkraft beaufschlagt, wobei die Spannvorrichtung einen drehbar an der Verlängerung (16) der einen Knochenschraube (1) gelagerten Schwenkhebel (37) aufweist, der im wesentlichen quer zur Längsrichtung der Verlängerung (16) verläuft und
an einem Anschlag (42) der anderen, dem zu reponierenden Wirbelkörper (50) zugeordneten Verlängerung (17) anliegt, **dadurch gekennzeichnet**, daß die Gelenkverbindungen (9, 10) zwischen Knochenschrauben (1, 2) und Querstrebe (11) fixierbar sind.

2. Fixateur nach Anspruch 1, dadurch gekennzeichnet, daß der Anschlag (42) an der Verlängerung (17) des zu reponierenden Wirbelkörpers (50) um eine Achse verschwenkbar ist, die zur Schwenkachse des Hebels (37) parallel verläuft.

3. Fixateur nach Anspruch 2, dadurch gekennzeichnet, daß der Anschlag (42) den Hebel (37) umgibt und relativ zu dem Hebel (37) in dessen Längsrichtung frei verschieblich ist.

4. Fixateur nach einem der voranstehenden Ansprüche, dadurch gekennzeichnet, daß der Hebel (37) im Bereich zwischen den beiden Verlängerungen (16, 17) ein Aussengewinde trägt, auf das eine Anschlagmutter (41) aufgeschraubt ist.

5. Fixateur nach einem der voranstehenden Ansprüche, dadurch gekennzeichnet, daß der Hebel (37) zweiarmig ausgebildet ist, wobei ein Arm (40) zu dem Anschlag (42) an der Verlängerung (17) des zu reponierenden Wirbelkörpers (50) führt, während der andere Arm (46) als Griffteil ausgeführt ist.

6. Fixateur nach einem der voranstehenden Ansprüche, dadurch gekennzeichnet, daß die Verlängerung (16), an der der Hebel (37) gelagert ist, einen festen Anschlag (49) trägt, gegen den ein am Hebel (37) verstellbar gelagertes Stellglied (48) anschlägt und dadurch ein Absinken des zu reponierenden Wirbelkörpers (50) verhindert.

7. Fixateur nach Anspruch 6, dadurch gekennzeichnet, daß das Stellglied (48) eine in ein Innengewinde (47) des Hebels (37) eingeschraubte, aus dem Innengewinde (47) hervorstehende Stellschraube ist.

8. Fixateur nach einem der voranstehenden Ansprüche, dadurch gekennzeichnet, daß die Verlängerungen (16, 17) lösbar mit den Knochenschrauben (1 beziehungsweise 2) verbunden sind.

9. Fixateur nach Anspruch 8, dadurch gekennzeichnet, daß die Verlängerungen (16, 17) Hülsen (18 beziehungsweise 19) aufweisen, an denen der Hebel (37) beziehungsweise der Anschlag (42) gelagert sind, und daß die Hülsen (18, 19) durch die Hülsen (18, 19) durchsetzende, in die Knochenschrauben (1 beziehungsweise 2) einschraubbare Fixierschrauben (20 beziehungsweise 21) an den Knochenschrauben (1 beziehungsweise 2) gehalten sind.

10. Fixateur nach einem der voranstehenden Ansprüche, dadurch gekennzeichnet, daß die Verlängerungen (16, 17) an ihren freien Enden Rücksprünge (32, 33 beziehungsweise 34, 35) zur Aufnahme eines Distraktionsinstrumentes tragen.

## Claims

1. An internal fixator for setting a lumbar spondylolisthesis having two bone screws (1, 2) screwable into the vertebral body to be set or an adjacent bone, respectively, and flexibly connected in their regions protruding from the bones by means of a cross-strut (11), having extensions (16, 17) holding the bone screws (1, 2) and running in the longitudinal direction thereof, and having a clamping device which acts on one of the two bone screws (1, 2) with a pulling force, wherein the clamping device has a pivoting lever (37) rotatably mounted on the extension (16) of one bone screw (1) and extending substantially transversely to the longitudinal direction of the extension (16), and resting on a stop (42) of the other extension (17) associated with the vertebral body (50) to be set, **characterised in that** the articulations (9, 10) between the bone screws (1, 2) and the cross-strut (11) are lockable.

2. A fixator according to Claim 1, **characterised in that** the stop (42) on the extension (17) of the vertebral body (50) to be set is pivotable about an axis which extends parallel to the axis of rotation of the lever (37).

3. A fixator according to Claim 2, **characterised in that** the stop (42) surrounds the lever (37) and is freely displaceable in relation to the lever (37) in the longitudinal direction thereof.

4. A fixator according to any one of the preceding claims, **characterised in that** the lever (37) has in the region between the two extensions (16, 17) an external thread, and a stop nut (41) is screwed thereon.

5. A fixator according to any one of the preceding claims, **characterised in that** the lever (37) is two-armed, wherein one arm (40) leads to the stop (42) on the extension (17) of the vertebral body (50) to be set, while the other arm (46) is shaped like a handle.

6. A fixator according to any one of the preceding claims, **characterised in that** the extension (16) on which the lever (37) is mounted has a stationary stop (49) against which an adjusting member (48), mounted on the lever so as to be adjustable, abuts and consequently prevents sinking of the vertebral body (50) to be set.

7. A fixator according to Claim 6, **characterised in that** the adjusting member (48) is an adjusting screw screwed into an internal thread (47) of the lever and protruding from the said internal thread (47).

8. A fixator according to any one of the preceding claims, **characterised in that** the extensions (16, 17) are detachably connected to the bone screws (1 and 2 respectively).

9. A fixator according to Claim 8, **characterised in that** the extensions (16, 17) have sleeves (18 and 19 respectively) on which the lever (37) and the stop (42) respectively are mounted, and the sleeves (18, 19) are held on the bone screws (1 and 2 respectively) by fixing screws (20 and 21 respectively) penetrating the sleeves (18, 19) and screwable in the bone screws (1 and 2 respectively).

10. A fixator according to any one of the preceding claims, **characterised in that** the free ends of the extensions (16, 17) have recesses (32, 33 and 34, 35 respectively) to receive a traction instrument.

## Revendications

1. Fixateur interne pour la réduction d'un spondylolisthésis lombaire, comprenant deux vis pour fracture osseuse (1, 2) pouvant être vissées respectivement dans le corps vertébral à repositionner et dans un os voisin, qui sont reliées de façon articulée entre elles par leurs parties dépassant des os au moyen d'une entretoise transversale (11), des rallonges (16, 17) attaquant les vis (1, 2) et s'étendant dans le sens de leur longueur, ainsi qu'un dispositif tendeur appliquant une force de traction à l'une des deux vis (1, 2) et comprenant un levier pivotant (37) monté rotatif sur la rallonge (16) d'une des vis (1), levier qui est essentiellement orienté transversalement à la direction longitudinale de la rallonge (16) et appliqué contre une butée (42) de l'autre rallonge (17), laquelle est coordonnée au corps vertébral (50) à repositionner, caractérisé en ce que les liaisons articulées (9, 10) entre les vis (1, 2) et l'entretoise transversale (11) sont blocables.

2. Fixateur selon la revendication 1, caractérisé en ce que la butée (42) est disposée pivotante, sur la rallonge (17) du corps vertébral (50) à repositionner, autour d'un axe parallèle à l'axe de pivotement du levier (37).

3. Fixateur selon la revendication 2, caractérisé en ce que la butée (42) entoure le levier (37) et est librement mobile en translation par rapport au levier (37) dans le sens de la longueur de celui-ci.

4. Fixateur selon une des revendications précédentes, caractérisé en ce que le levier (37) porte, dans la zone entre les deux rallonges (16, 17), un filetage extérieur sur lequel est vissé un écrou de butée (41).

5. Fixateur selon une des revendications précédentes, caractérisé en ce que le levier (37) possède deux bras dont l'un (40) mène à la butée (42) sur la rallonge (17) coordonnée au corps vertébral (50) à repositionner, tandis que l'autre bras (46) est réalisé à la façon d'un manche.

6. Fixateur selon une des revendications précédentes, caractérisé en ce que la rallonge (16) sur laquelle est monté le levier (37), porte une butée fixe (49) contre laquelle s'applique un élément de réglage (48) disposé ajustable sur le levier (37), et empêche ainsi l'abaissement du corps vertébral (50) à repositionner.

7. Fixateur selon la revendication 6, caractérisé en ce que l'élément de réglage (48) est une vis de réglage vissée dans un filetage intérieur (47) du levier (37) et dépassant de ce filetage (47).

8. Fixateur selon une des revendications précédentes, caractérisé en ce que les rallonges (16, 17) sont reliées de façon amovible aux vis (1 et 2 respectivement).

9. Fixateur selon la revendication 8, caractérisé en ce que les rallonges (16, 17) comportent des douilles (18 et 19 respectivement) sur lesquelles sont montés respectivement le levier (37) et la butée (42), et que les douilles (18, 19) sont maintenues en place sur les vis pour fracture osseuse (1 et 2 respectivement) par des vis de fixation (20 et 21 respectivement) qui traversent les douilles (18, 19) et peuvent être vissées dans les vis pour fracture osseuse (1 et 2).

10. Fixateur selon une des revendications précédentes, caractérisé en ce que les rallonges (16, 17) présentent à leurs extrémités libres des rentrants (32, 33 et 34, 35 respectivement) pour l'application d'un instrument de distension ou d'écartement.
